# EUROPEAN PATENT APPLICATION

(11) **EP 3 165 250 A1**
(43) Date of publication of application: **10.05.2017**
(21) Application number: 15193115.1
(22) Date of filing: 05.11.2015
(51) Int. Cl.: A61M 16/04, A61M 39/04

(54) **CONNECTING BASE FOR SUCTION CATHETERS**

(71) Applicant: Vitaltec Corporation, Taichung City (TW)
(72) Inventor: HSIAO, Hui-Ling, Taichung City (TW); CHIU, Sheng-Yu, Taichung City (TW)
(74) Representative: Viering, Jentschura & Partner mbB Patent- und Rechtsanwälte

(57) **Abstract**

A connecting base for suction catheters has a body (20, 20A, 20B, 20C) and a check valve (30, 30B). The body (20, 20A, 20B, 20C) has an assembling portion (21, 21A, 21B, 21C) and a jointing tube (22, 22A, 22B, 22C). The jointing tube (22, 22A, 22B, 22C) is located in the assembling portion (21, 21A, 21B, 21C). The check valve (30, 30B) has a positioning ring (31, 31A, 31B) and two valve blades (32, 32A, 32B, 32C). The positioning ring (31, 31A, 31B) engages with the assembling portion (21, 21A, 21B, 21C), and the two valve blades (32, 32A, 32B, 32C) respectively abut an edge of a positioning hole (222) of the jointing tube (22, 22A, 22B, 22C). The two valve blades (32, 32A, 32B, 32C) may close automatically to increase the airtight effect of the connecting base (10).

## Description

### 1. Field of the Invention

The present invention relates to a connecting device, and more particularly to a connecting base with a check valve for suction catheters that can improve a positioning function of the suction catheters.

### 2. Description of Related Art

Some patients cannot breathe by themselves after a surgery, the secretions and the phlegm may block the tracheas of the patients, so a conventional suction catheter is used to draw the secretions and the phlegm from the trachea. The conventional suction catheter has a connecting base and a catheter. The connecting base is connected to a communicating tube that is connected with the tracheas of the patient, and has a positioning segment. The positioning segment is formed in the connecting base. The catheter has a proximal end. The proximal end of the catheter is inserted in the connecting base to draw the phlegm in the tracheas of the patient, and is mounted through and abuts the positioning segment of the connecting base securely. The proximal end of the catheter can continually draw the phlegm after the catheter moves back from the trachea of the patient to the connecting base, and this can reduce the uncomfortable feeling of the patient during the drawing process, and the catheter can continually draw the phlegm in the connecting base.

However, the connecting base of the conventional suction catheter does not have any valve blade, and this makes the connecting base not airtight. In the process of moving the catheter back to the connecting base and drawing the phlegm, the drawing effect of the catheter is not good since an outer surrounding surface of the catheter and the positioning segment of the connecting base are not airtight. Then, the phlegm drawing effect of the catheter is not good, and the phlegm may flow back to the trachea of the patient and the phlegm may infect the patient again.

US Patent No. 7,191,782 is provided and entitled "Respiratory suction catheter apparatus configured for releasable attachment with an artificial airway structure". In the US Patent, a connecting base has a valve and an annual ring, the valve is a single flap and is pivotally attached to the annular ring, and the annual ring is engaged in an interior of the connecting base. So the connecting base is separated from the trachea of the patient, and this can reduce the chance that the phlegm flows back to the trachea of the patient.

However, the valve is only pivotally attached to the annular ring, so the abutting force between the valve and the annual ring is inadequate. A gap is easily formed between the valve and the annual ring when the catheter moves back to the connecting base, and this makes the airtight effect of the valve inadequate, and the phlegm still may flow back to the trachea of the patient. Therefore, the conventional connecting base for the suction catheters should be improved.

To overcome the shortcomings of the connecting base for the conventional suction catheters, the present invention provides a connecting base for suction catheters to mitigate or obviate the aforementioned problems.

The main objective of the present invention is to provide a connecting base with a check valve for suction catheters, which can improve a positioning function of the suction catheters.

The connecting base for suction catheters has a body and a check valve. The body has an assembling portion and a jointing tube. The jointing tube is located in the assembling portion. The check valve has a positioning ring and two valve blades. The positioning ring engages with the assembling portion, and the two valve blades respectively abut an edge of a positioning hole of the jointing tube. The two valve blades may close automatically to increase the airtight effect of the connecting base.

Other objects, advantages, and novel features of the invention will become more apparent from the following detailed description when taken in conjunction with the accompanying drawing.

### IN THE DRAWINGS:

Fig. 1 is an operational perspective view of a first embodiment of a connecting base for suction catheters in accordance with the present invention;
Fig. 2 is an enlarged perspective view of the connecting base in Fig. 1;
Fig. 3 is an exploded perspective view of the connecting base in Fig. 2;
Fig. 4 is a perspective view in partial section of the connecting base in Fig. 2;
Fig. 5 is a cross sectional side view of the connecting base in Fig. 2;
Fig. 6 is an operational cross sectional side view of the connecting base in Fig. 2;
Fig. 7 is a perspective view of a second embodiment of a connecting base in accordance with the present invention;
Fig. 8 is an exploded perspective view of the connecting base in Fig. 7;
Fig. 9 is an operational cross sectional side view of the connecting base in Fig. 7;
Fig. 10 is a perspective view of a third embodiment of a connecting base in accordance with the present invention;
Fig. 11 is an exploded perspective view of the connecting base in Fig. 10;
Fig. 12 is an operational cross sectional side view of the connecting base in Fig. 10;
Fig. 13 is an operational perspective view of the connecting base in Fig. 10;
Fig. 14 is a perspective view of a fourth embodiment of a connecting base in accordance with the present invention; and
Fig. 15 is an operational cross sectional side view of the connecting base in Fig. 14.

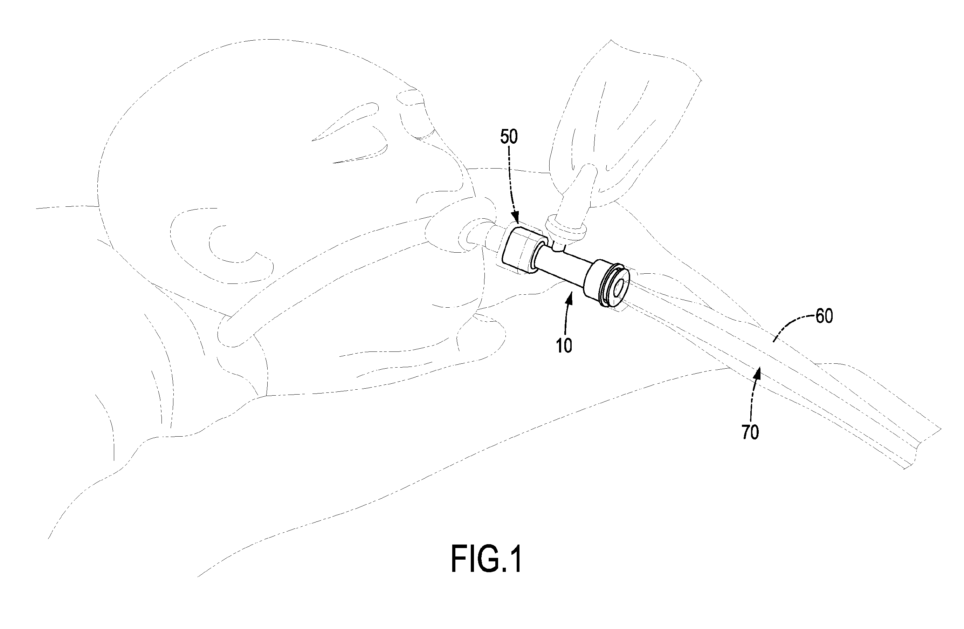

With reference to Figs. 1 to 3, a first embodiment of a connecting base 10 for suction catheters in accordance with the present invention is connected to a communicating tube 50 and a protective sleeve 60, and a catheter 70 is mounted through the protective sleeve 60 and the connecting base 10. The connecting base 10 comprises a body 20 and a check valve 30.

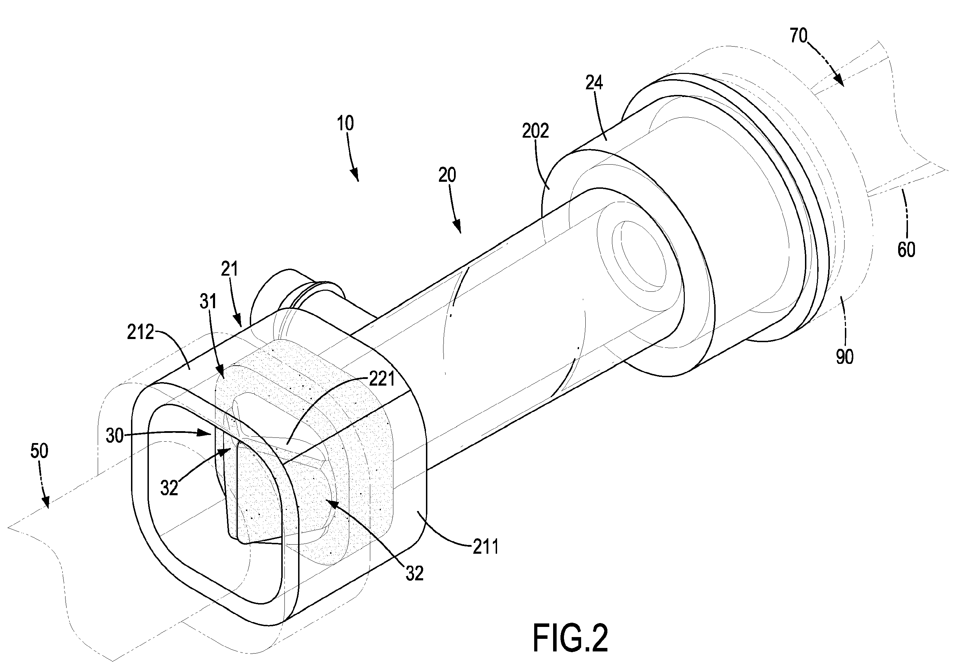

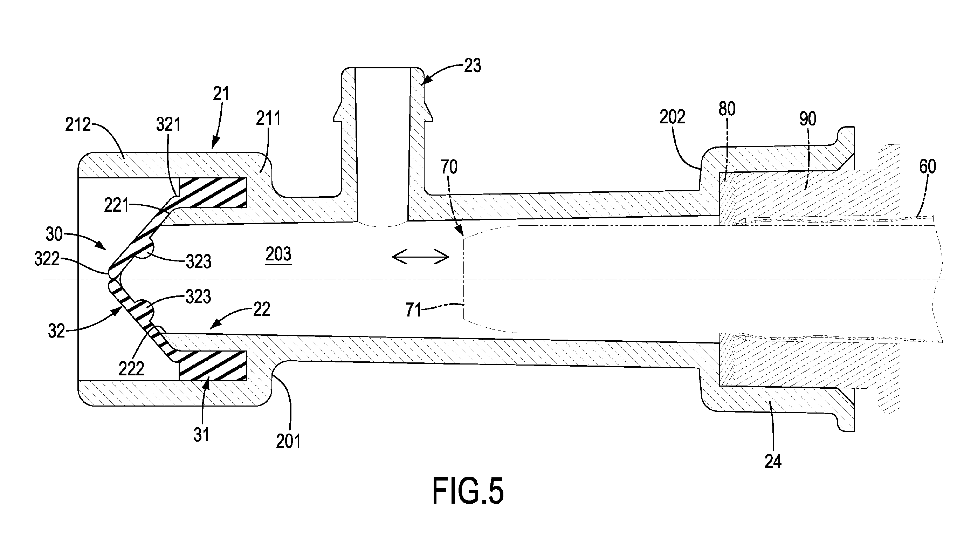

With reference to Figs. 2 to 4, the body 20 is a circular transparent tube and has two opening edges, a surrounding surface, an axis, a mounting ring edge 201, an engaging ring edge 202, a receiving space 203, an assembling portion 21, a jointing tube 22, a vent port 23, and an engaging portion 24. With reference to Fig. 5, the mounting ring edge 201 and the engaging ring edge 202 are formed on the two opening edges of the body 20 respectively. The receiving space 203 is formed in the body 20 between the mounting ring edge 201 and the engaging ring edge 202 of the body 20. The assembling portion 21 is a polygonal cover, is formed on and protrudes radially from the mounting ring edge 201, and has an opening, an inner surrounding surface, a base flange 211, and a surrounding ring 212. The opening of the assembling portion 21 faces away from the engaging ring edge 202 of the body 20. The base flange 211 is erect and is formed with the body 20, and has an inner side surface and an outer surrounding surface. The surrounding ring 212 is disposed horizontally and is formed with the base flange 211, and has an inner surrounding surface and an outer surrounding surface. Preferably, the assembling portion 21 is a square cover.

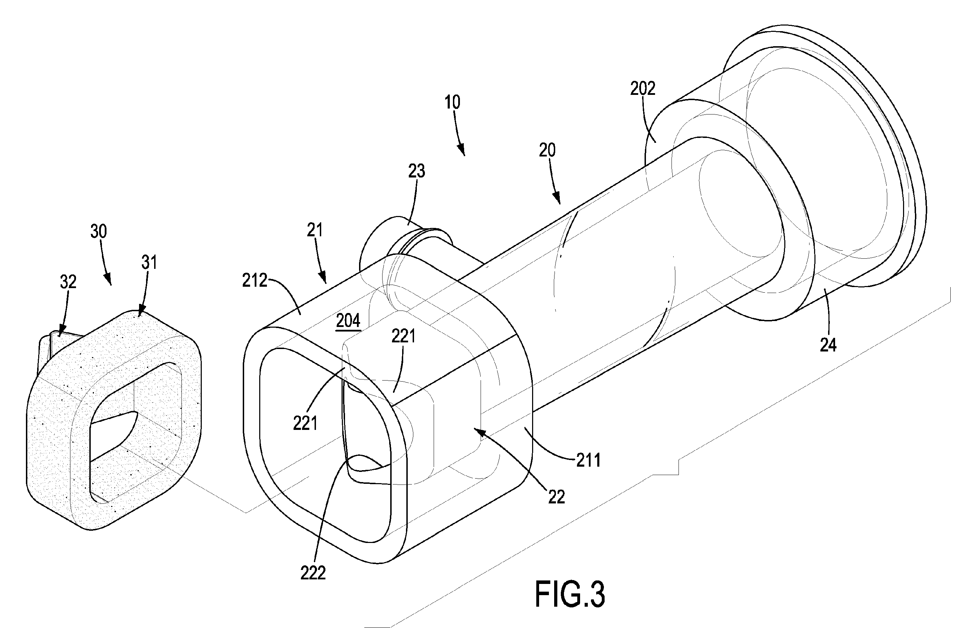

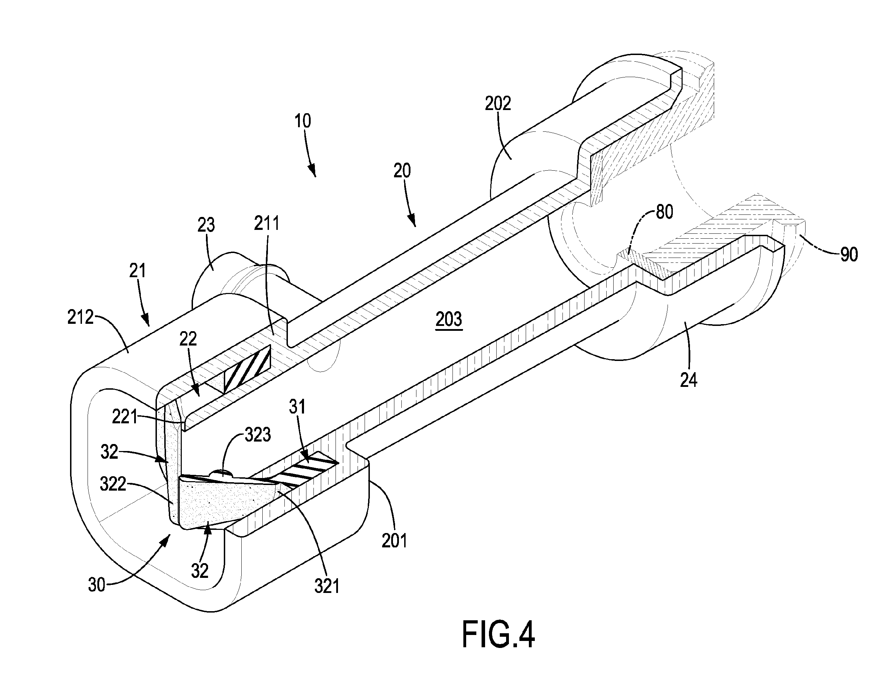

With reference to Figs. 3 and 4, the jointing tube 22 is a polygonal tube and protrudes axially from the mounting ring edge 201 toward the opening of the assembling portion 21. The jointing tube 22 is formed in the assembling portion 21 and has an outer surrounding surface, a distal end, two supporting portions 221, and a positioning hole 222. Preferably, the jointing tube is a square tube. A mounting chamber 204 is formed between the inner surrounding surface of the assembling portion 21 and the outer surrounding surface of the jointing tube 22. The distal end of the jointing tube 22 is away from the base flange 211. The two supporting portions 221 are disposed at the distal end of the jointing tube 22. The two supporting portions 221 are integrated with an end of the jointing tube 22 which is opposite to the base flange 211 to form a conical shape. Each one of the two supporting portions 221 has an opening edge. The positioning hole 222 is formed through the opening edges of the two supporting portions 221 and is formed through the distal end of the jointing tube 22. The positioning hole 222 is U-shaped and has an edge. The vent port 23 protrudes from the surrounding surface of the body 20 and is adjacent to the assembling portion 21. The vent port 23 is a tube and communicates with the receiving space 203 of the body 20. The engaging portion 24 is a circular cover and protrudes radially from the engaging ring edge 202, and has an opening. The opening of the engaging portion 24 faces away from the mounting ring edge 201.

With reference to Figs. 3 to 5, the check valve 30 is mounted around the jointing tube 22 of the body 20, engages with the assembling portion 21, and is located in the mounting chamber 204. The check valve 30 has a positioning ring 31 and two valve blades 32. The shape of the positioning ring 31 is corresponding to the shapes of the assembling portion 21 and the jointing tube 22. The positioning ring 31 has an outer surrounding surface, an inner surrounding surface, and a side surface. The outer surrounding surface of the positioning ring 31 abuts the inner surrounding surface of the surrounding ring 212 of the assembling portion 21. The inner surrounding surface of the positioning ring 31 abuts the outer surrounding surface of the jointing tube 22. The side surface of the positioning ring 31 is adjacent to the mounting ring edge 201 and contacts the inner side surface of the base flange 211 of the assembling portion 21, and this makes the positioning ring 31 positioned between the assembling portion 21 and the jointing tube 22.

The two valve blades 32 are formed with the inner surrounding surface of the positioning ring 31 and abut the edge of the positioning hole 222 of the jointing tube 22 respectively between the two supporting portions 221. Each one of the two valve blades 32 has an inner surface, an engaging edge 321, two side edges, a free edge 322, and a scraping protrusion 323. The engaging edge 321 of the valve blade 32 is formed with the inner surrounding surface of the positioning ring 31. The two side edges of the valve blade 32 respectively abut the two opening edges of the two supporting portions 221.

The free edge 322 of the valve blade 32 is toward the axis of the body 20, thereby the two valve blades 32 incline toward the axis of the body 20. So the free edges 322 of the two valve blades 32 abut each other closely, and a sealing force is formed between the two valve blades 32. The two valve blades 32 are formed in an inverted-V shape, and the check valve 30 is formed as a duckbill valve. The two valve blades 32 are closed automatically when the two valve blades 32 are free from external forces. The scraping protrusion 323 protrudes inwardly from the inner surface of the valve blade 32, and the scraping protrusion 323 is a circular protrusive point. The positioning hole 222 of the jointing tube 22 between the two supporting portions 221 is a U-shaped hole, and this makes the shape of the two valve blades 32 match the shape of the positioning hole 222, thereby the two side edges of the two valve blades 32 abut the opening edges of the two supporting portions 221 tightly.

With reference to Figs. 1, 5, and 6, the first embodiment of the present invention is assembled with a suction catheter and has the following operation procedures.

First, the engaging portion 24 of the body 20 is connected to the protective sleeve 60 via a phlegm scraping slice 80 and a locating ring 90. The phlegm scraping slice 80 is disposed on an inner surrounding surface of the engaging portion 24, and the locating ring 90 is mounted around the protective sleeve 60 and abuts the inner surrounding surface of the engaging portion 24.A catheter 70 is inserted into the receiving space 203 via the engaging portion 24. The catheter 70 has a surrounding surface, a proximal end 71, and a distal end. The surrounding surface of the catheter 70 engages with the phlegm scraping slice 80. The vent port 23 of the body 20 communicates with a saline bag, and keeps the saline bag un-pressed, and the receiving space 203 of the body 20 is held in a sealed state. The assembling portion 21 of the body 20 is connected to the communicating tube 50 on a patient, and the distal end of the catheter 70 is connected to a vacuum device.

Second, one hand of a user holds the connecting base 10, and the other hand of the user holds the protective sleeve 60, and moves the proximal end 71 of the catheter 70 into a proper depth in the patient's trachea. At the same time, the proximal end 71 of the catheter 70 stretches through the jointing tube 22 and pushes the two valve blades 32 of the check valve 30, and this makes the communicating tube 50 communicate with the jointing tube 22 via the check valve 30.

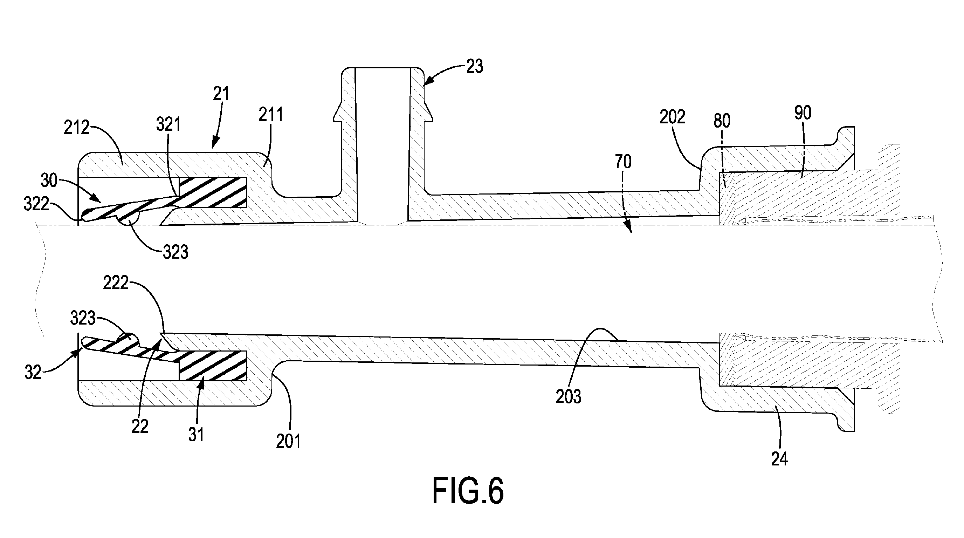

Third, by turning on the vacuum device, the vacuum device can provide a suction force to the catheter 70, and secretions and phlegm of the patient may be removed by the catheter 70. The hand that holds the protective sleeve 10 keeps holding the protective sleeve 10, and then withdraws the catheter 70 slowly, and this makes the proximal end 71 of the catheter 70 move back into the receiving space 203 of the body 20. With reference to Fig. 6, during the procedure of withdrawing the catheter 70, the catheter 70 stretches through a gap between the two valve blades 32, and this makes the two valve blades 32 stay open. And the scraping protrusions 323 of the two valve blades 32 would scrape off the secretions and the phlegm that are attached on the catheter 70. The free edges 322 of the two valve blades 32 and the surrounding surface of the catheter 70 are disposed at a spaced interval, and this makes the secretions and the phlegm that are scraped by the two valve blades 32 stay in the inner surfaces of the two valve blades 32. The two valve blades 32 of the check valve 30 would close automatically after the proximal end 71 of the catheter 70 is moved back to the receiving space 203 of the body 20 by the user.

Fourth, after the proximal end 71 of the catheter 70 moves back to the receiving space 203 of the body20, saline solution in the saline bag flows in the receiving space 203 via the vent port 23 when the secretions and the phlegm are too thick. The patient's secretions and phlegm become thinner, such that the catheter 70 can withdraw the patient's secretions and phlegm from the connecting base 10. The two valve blades 32 of the check valve 30 would close automatically after the proximal end 71 of the catheter 70 are moved back to the receiving space 203 of the body 20 and the receiving space 203 is at a sealed state, so the effect of withdrawing secretions and phlegm can be maintained. Additionally, the two valve blades 32 of the check valve 30 can prevent the secretions and the phlegm from flowing back to the patient via the jointing tube 22.

In the present invention, the assembling portion 21, the jointing tube 22, and the positioning ring 31 are designed in square shapes, and this makes the check valve 30 positioned with the body 20 easily. The two valve blades 32 of the check valve 30 are inverted-V-shaped, and the two valve blades 32 abut the two opening edges of the two supporting portions 221 of the jointing tube 22 respectively, and the check valve 30 is formed as a duckbill valve. The two supporting portions 221 are not integrated with the side edges of the two valve blades 32, so the catheter 70 can push the two valve blades 32 smoothly, and this makes the communicating tube 50 communicate with the jointing tube 22. The two valve blades 32 may close automatically after the catheter 70 moves back to the receiving space 203, thereby the receiving space 203 remains sealed, and the phlegm can be withdrawn. Additionally, in the procedure of moving the catheter 70 back to the receiving space 203, the scraping protrusion 323 of each valve blade 32 continually abuts the outer surrounding surface of the catheter 70, and the scraping protrusion 323 of each valve blade 32 would scrape off the secretions and the phlegm attached on the catheter 70. The secretions and the phlegm attached on the outer surrounding surface of the catheter 70 would stick to the inner surface of each valve blade 32, and the vent port 23 can be used to wash the inner surface of each valve blade 32. Therefore, the secretions and the phlegm can be preventing from sticking between the free edge 322 of each valve blade 32, the surrounding ring 212 and the check valve 30, and this can prevent the surrounding ring 212 and the check valve 30 from being over polluted.

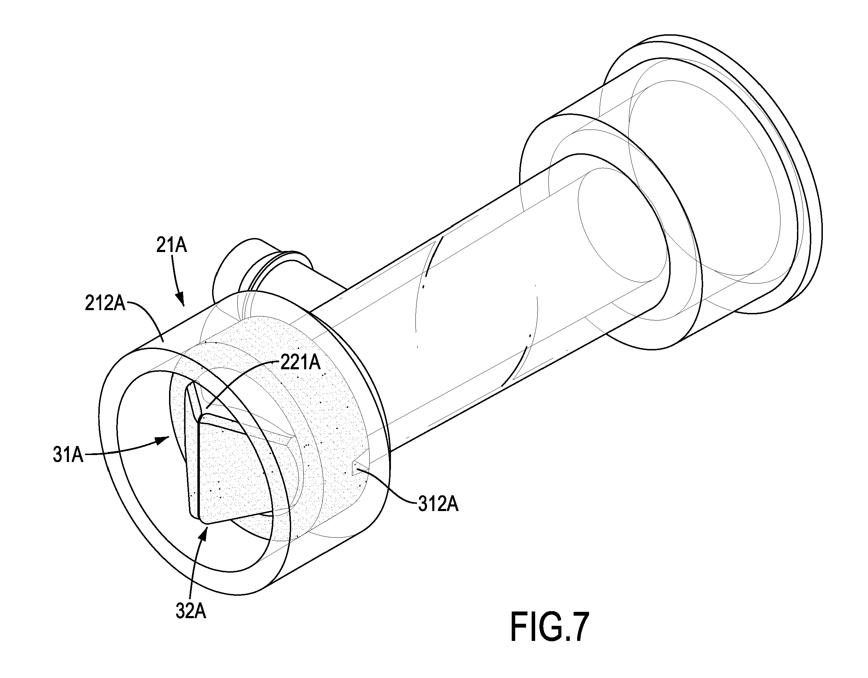

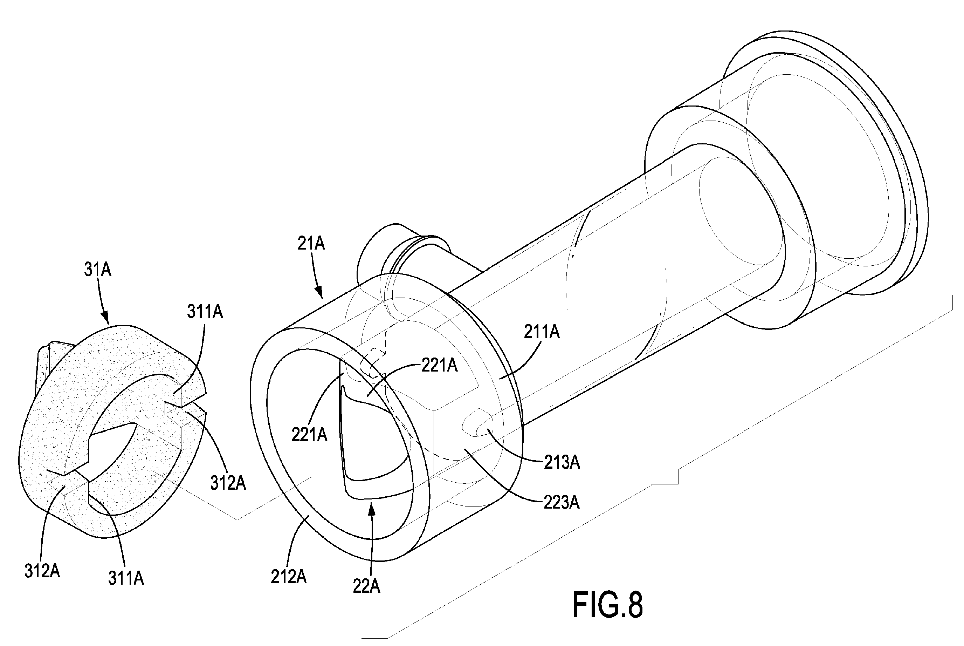

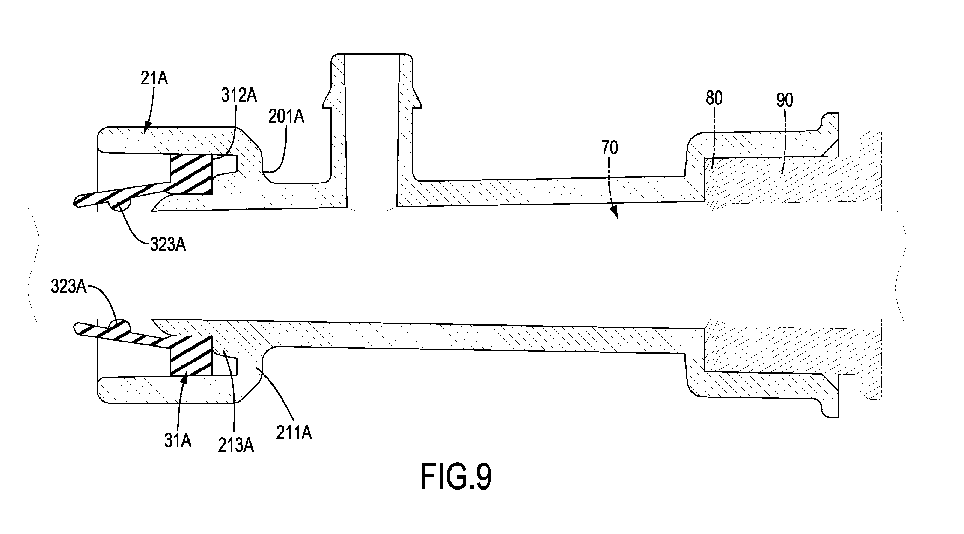

With reference to Figs. 7 to 9, a second embodiment of a connecting base for suction catheters in accordance with the present invention is similar to the first embodiment, and the differences are: the assembling portion 21A is a circular cover and has two positioning ribs 213A. The two positioning ribs 213A protrude from the inner side surface of the base flange 211 A toward the opening of the assembling portion 21. The two positioning ribs 213A are aligned in a radial line of the base flange 211A. The jointing tube 22A is a circular tube and has two contacting faces 223A, and each one of the two supporting portions 221A is conical-shaped. The two contacting faces 223A are formed on the outer surrounding surface of the jointing tube 22A, the two contacting faces 223A are aligned with each other, and each one of the two contacting faces 223A is a plane. The two positioning ribs 213A are formed with the two contacting faces 223A respectively. The positioning ring 31A has two positioning faces 311A and two mounting recesses 312A. The two positioning faces 311A are formed on the inner surrounding surface of the positioning ring 31A. The positions and the shapes of the two positioning faces 311A are corresponding to the positions and the shapes of the two contacting faces 223A respectively, and this makes the two positioning faces 311A abut the two contacting faces 223A of the jointing tube 22A respectively. The two mounting recesses 312A are formed in the side surface of the positioning ring 31A. The shapes and the positions of the two mounting recesses 312A are corresponding to the shapes and positions of the two positioning ribs 213A, and the two positioning ribs 213A engage with the two mounting recesses 312A respectively. With reference to Fig. 9, the operation procedure of the second embodiment is the same as the first embodiment, and detailed description thereof will be omitted.

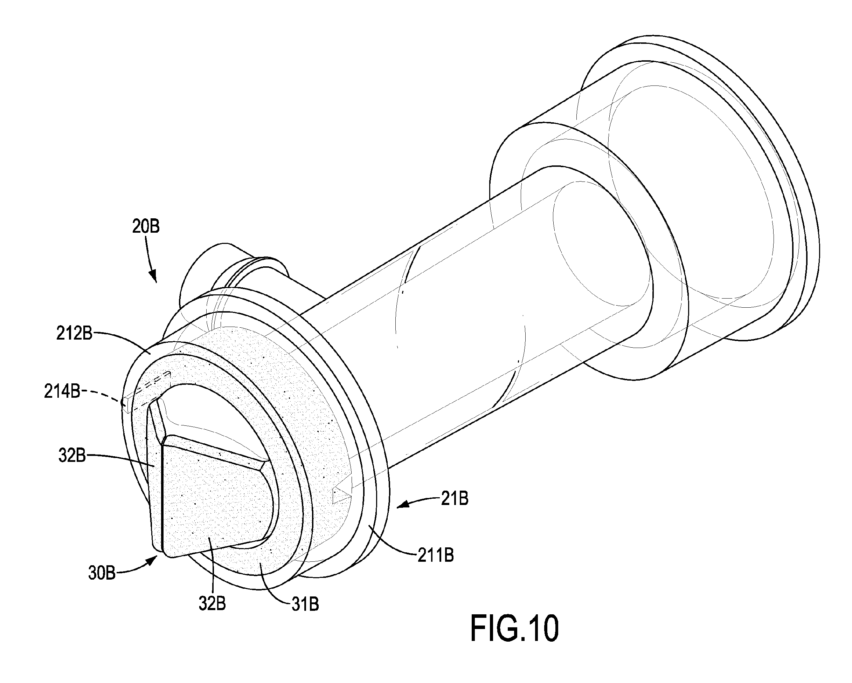

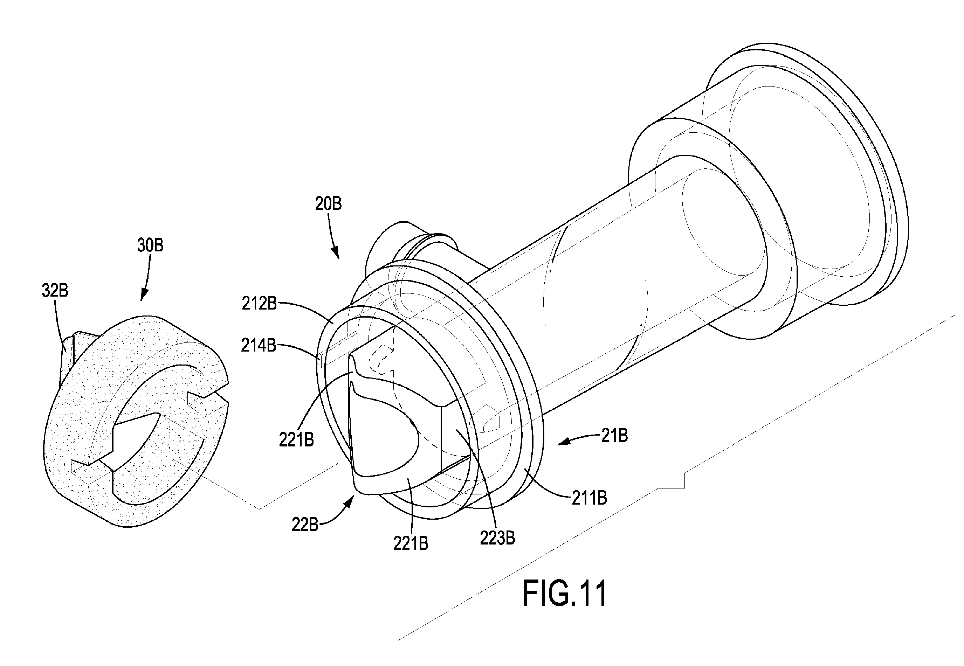

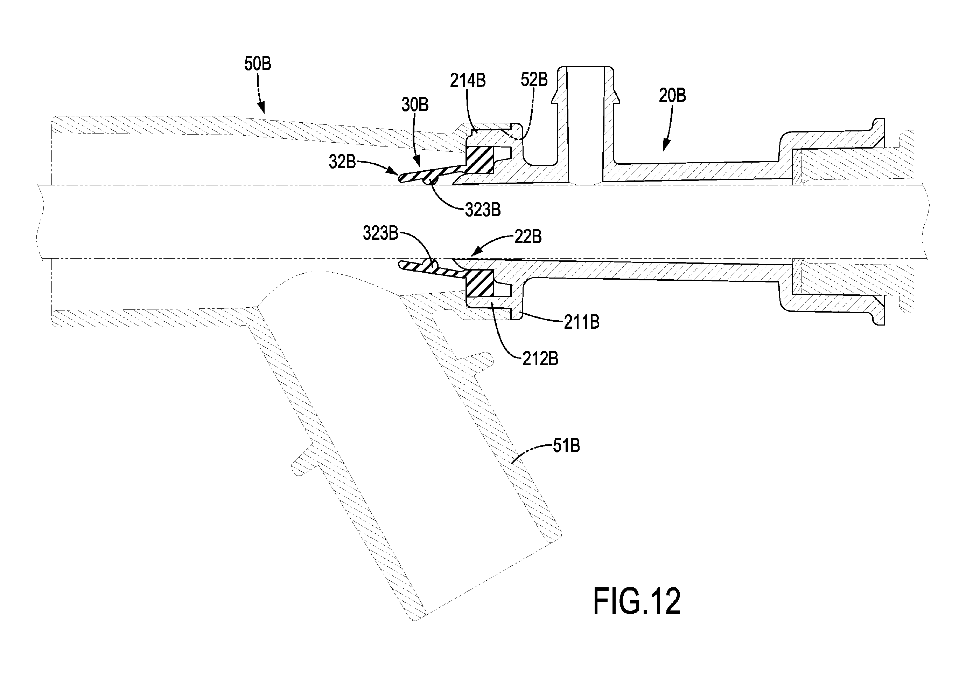

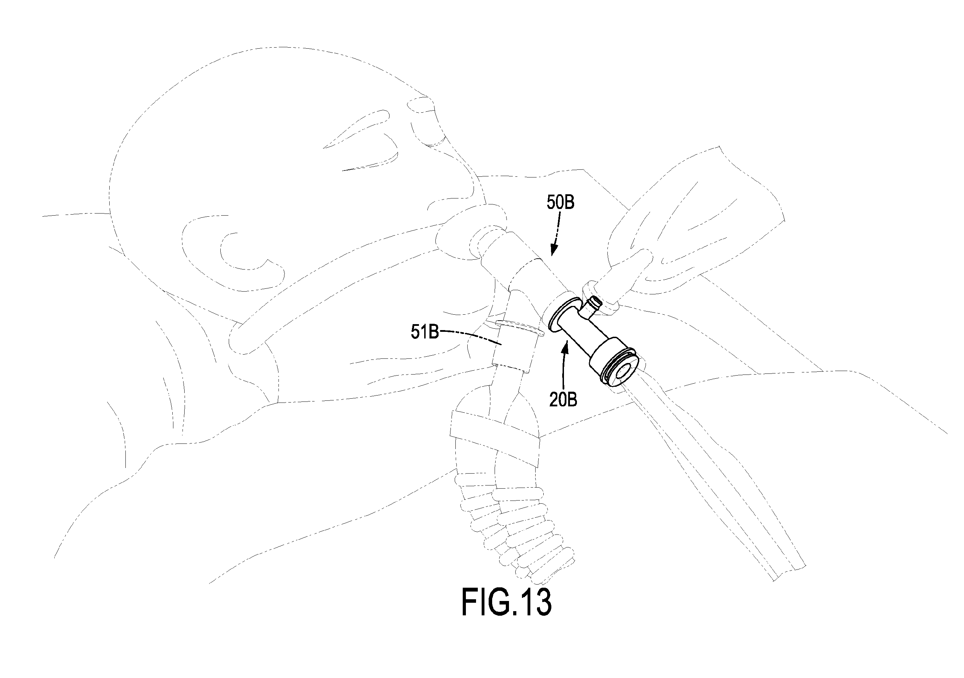

With reference to Figs. 10 to 12, a third embodiment of a connecting base for suction catheters in accordance with the present invention is similar to the second embodiment, and the differences are: the outer surrounding surface of the base flange 211B of the assembling portion 21B sticks radially out of the outer surrounding surface of the surrounding ring 212B. The surrounding ring 212B has a combining rib 214B. The combining rib 214B is formed on and protrudes radially from the outer surrounding surface of the surrounding ring 212B. The two supporting portions 221B of the jointing tube 22B stick out of a front edge of the assembling portion 21B, so the two valve blades 32 stick out of the front edge of the assembling portion 21B. With reference to Figs. 12 and 13, the operation procedure of the third embodiment is similar to the first embodiment, the differences are: the communicating tube 50B has a distal end, an outer surrounding surface, an inner surrounding surface, an interior, a linking tube 51B, and a combining recess 52B. The linking tube 51B is formed on the outer surrounding surface of the communicating tube 50B and communicates with the interior of the communicating tube 50B. The distal end of the communicating tube 50B is adjacent to the linking tube 51B. The combining recess 52B is radially formed in the inner surrounding surface of the communicating tube 50B and adjacent to the distal end of the communicating tube 50B. The distal end of the communicating tube 50B is mounted around the assembling portion 21B of the body 20B, and the combining recess 52B of the communicating tube 50B engages with the combining rib 214B of the assembling portion 21B. So the communicating tube 50B can engage with the body 20B, and the linking tube 51B of the communicating tube 50B can be connected with a ventilation machine.

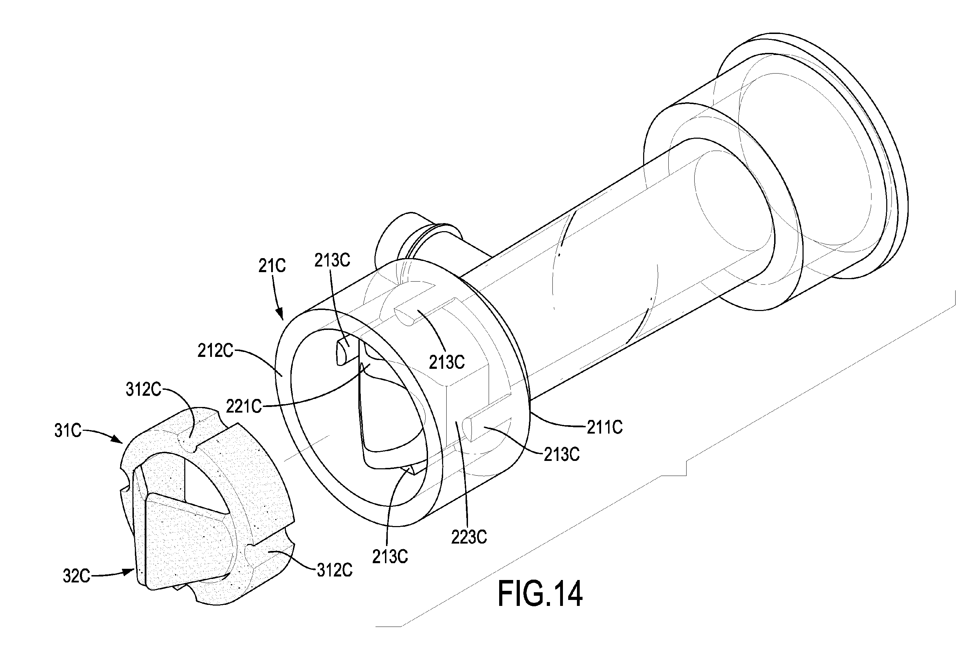

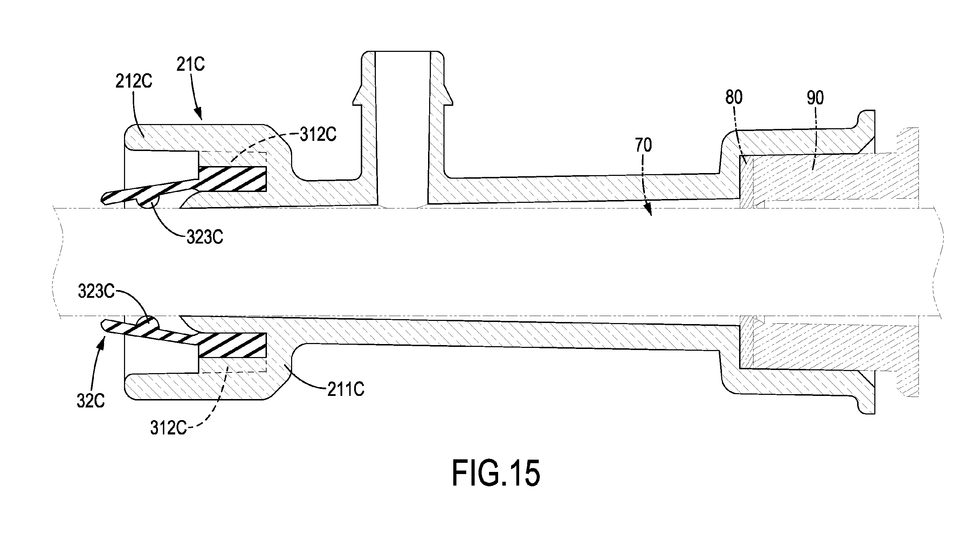

With reference to Figs. 14 and 15, a fourth embodiment of a connecting base for suction catheters in accordance with the present invention is similar to the second embodiment, and the differences are: the assembling portion 21C has four positioning ribs 213C. The four positioning ribs 213C are formed on and protrude axially from the inner side surface of the base flange 211C and protrude toward the opening of the assembling portion 21C. The four positioning ribs 213C are respectively disposed at the 0° ,90° ,180° , and 270° positions of the base flange 211C, and the four positioning ribs 213C are integrated with the inner surrounding surface of the surrounding ring 212C. The positioning ring 31C has four mounting recesses 312C, and the four mounting recesses 312C are radially formed in the outer surrounding surface of the positioning ring 31C. Each one of the four mounting recesses 312C is axially formed through the outer surrounding surface of the positioning ring 31C. The shapes and the positions of the four mounting recesses 312C are corresponding to the shapes and positions of the four positioning ribs 213C. With reference to Fig. 15, the operation procedure of the fourth embodiment is the same as the second embodiment, and detailed description thereof will be omitted.

According to the above-mentioned features and structural relationships of the present invention, the assembling portion 21, 21A, 21B, 21C, the jointing tube 22, 22A and the positioning ring 31, 31A, 31B are respectively designed in polygonal shapes, and the position ribs 213A, 213C are designed to engage with the mounting recesses 312A, 312C. Therefore, the check valve 30, 30B can be assembled with and positioned with the body 20, 20B easily to ensure the two valve blades 32, 32B of the check valve 30, 30B abut the two supporting portions 221, 221A, 221B of the jointing tube 22, 22A, 22B respectively, and the positioning effect of the check valve 30, 30B can be maintained. The two valve blades 32, 32B of the check valve 30, 30B and the two supporting portions 221, 221A, 221B of the jointing tube 22, 22A, 22B are separate elements that abut each other, so the two valve blades 32, 32B can be easily opened and remain airtight sealed when in a closed state, and the catheter 70 can draw the phlegm smoothly.

Additionally, the scraping protrusion 323, 323A, 323B, 323C of each valve blade 32, 32B would scrape off the secretions and the phlegm attached on the catheter 70. The secretions and the phlegm attached on the outer surrounding surface would stick on the inner surface of each valve blade 32, 32B, and the vent port 23 can be used to wash the inner surface of each valve blade 32, 32B. Therefore, the secretions and the phlegm can be prevented from sticking between the free edge 322 of each valve blade 32, 32B, the surrounding ring 212, 212B, 212C and the check valve 30, 30B, and this can prevent the surrounding ring 212, 212B, 212C and the check valve 30, 30B from being over polluted.

## Claims

1. A connecting base for suction catheters, **characterized in that** the connecting base (10) comprises:
a body (20, 20A, 20B, 20C) being a tube and having
an axis;
two opening edges;
a mounting ring edge (201) formed on one of the two opening edges of the body (20);
an engaging ring edge (202) formed on the other opening edge of the body (20);
a receiving space (203) formed in the body (20, 20A, 20B, 20C) between the mounting ring edge (201) and the engaging ring edge (202);
an assembling portion (21, 21A, 21B,21C) being a cover, disposed at the mounting ring edge (201), and having an inner surrounding surface and an opening, the opening of the assembling portion (21, 21A, 21B,21C) facing away from the engaging ring edge (202) of the body (20, 20A, 20B, 20C); and
a jointing tube (22, 22A, 22B, 22C) protruding axially from the mounting ring edge (201) toward the opening of the assembling portion (21, 21A, 21B,21C), the jointing tube (22, 22A, 22B, 22C) formed in the assembling portion (21, 21A, 21B, 21C) and having
an outer surrounding surface;
a distal end away from the mounting ring edge (201);
two supporting portions (221, 221A, 221B, 221C) disposed at the distal end of the jointing tube (22, 22A, 22B, 22C) and forming a conical shape;
a positioning hole (222) formed through the two supporting portions (221, 211 A, 211B, 211C) and formed through the distal end of the jointing tube (22, 22A, 22B, 22C), being U-shaped, and having an edge; and
a mounting chamber (204) formed between the inner surrounding surface of the assembling portion (21, 21A, 21B,21C) and the outer surrounding surface of the jointing tube (22, 22A, 22B, 22C); and
a check valve (30, 30B) mounted around the jointing tube (22, 22A, 22B, 22C) of the body (20, 20B), engaging with the assembling portion (21, 21A, 21B, 21C), and having
a positioning ring (31, 31A, 31B) engaging with the assembling portion (21, 21A, 21B, 21C), being in the mounting chamber (204), and having an inner surrounding surface, the shape of the positioning ring (31, 31A, 31B) corresponding to the shapes of the assembling portion (21, 21A, 21B, 21C) and the jointing tube (22, 22A, 22B, 22C); and
two valve blades (32, 32A, 32B, 32C) formed with the inner surrounding surface of the positioning ring (31, 31A, 31B) and abutting the edge of the positioning hole (222) of the jointing tube (22, 22A, 22B, 22C) respectively between the two supporting portions (221, 221A, 221B, 221C), and the two valve blades (32, 32A, 32B, 32C) inclining toward the axis of the body (20, 20A, 20B, 20C).

2. The connecting base for suction catheters as claimed in claim 1,
wherein the assembling portion (21) is a polygonal cover, and the jointing tube (22) is a polygonal tube.

3. The connecting base for suction catheters as claimed in claim 2,
wherein the assembling portion (21) is a square cover, and the jointing tube (22) is a square tube.

4. The connecting base for suction catheters as claimed in claim 3,
wherein each one of the two valve blades (32) has an inner surface and a scraping protrusion (323), the scraping protrusion (323) protrudes inwardly from the inner surface of the valve blade (32), and the scraping protrusion (323) is a circular protrusive point.

5. The connecting base for suction catheters as claimed in claim 1,
wherein
the assembling portion (21A, 21B) is a circular cover and protrudes radially from the mounting ring edge (201) and has
a base flange (211A, 211B) being erect and formed with the body (20A, 20B), and having an inner side surface and an outer surrounding surface;
a surrounding ring (212A, 212B) disposed horizontally and formed with the base flange (211A, 211B); and
two positioning ribs (213A, 213B) protruding from the inner side surface of the base flange (211A, 211B) toward the opening of the assembling portion (21A, 21B);
the jointing tube (22A, 22B) is a circular tube and has
two contacting faces (223A, 223B) formed on the outer surrounding surface of the jointing tube (22A, 22B), and each one of the two contacting faces (223A, 223B) being a plane;
the positioning ring (31A, 31B) has
two positioning faces (311A, 311B) formed on the inner surrounding surface of the positioning ring (31A, 31B), the position and the shape of the two positioning faces (311A, 311B) corresponding to the positions and the shapes of the two contacting faces (223A, 223B), and the two positioning faces (311A, 311B) abutting the two contacting faces (223A, 223B) of the jointing tube (22A, 22B) respectively; and
two mounting recesses (312A, 312B) formed in a side surface of the positioning ring (31A, 31B), the shapes and the positions of the two mounting recesses (312A, 312B) corresponding to the shapes and positions of the two positioning ribs (213A, 213B), and the two positioning ribs (213A, 213B) engaging with the two mounting recesses (312A, 312B) respectively.

6. The connecting base for suction catheters as claimed in claim 5, wherein
the outer surrounding surface of the base flange (211B) of the assembling portion (21B) sticks radially out of an outer surrounding surface of the surrounding ring (212B);
the surrounding ring (212B) has a combining rib (214B) protruding radially from the outer surrounding surface of the surrounding ring (212B); and
the two supporting portions (221B) of the jointing tube (22B) stick out of a front edge of the assembling portion (21B), and the two valve blades (32B) stick out of the front edge of the assembling portion (21B).

7. The connecting base for suction catheters as claimed in claim 5 or 6, wherein
the two positioning ribs (213A, 213B) are aligned in a radial line of the base flange (211A, 211B); and
the two contacting faces (223A, 223B) are aligned, each one of the two positioning ribs (213A, 213B) is formed with one of the two contacting faces (223A, 223B).

8. The connecting base for suction catheters as claimed in claim 7, wherein each one of the two valve blades (32A, 32B) has an inner surface and a scraping protrusion (323A, 323B), the scraping protrusion (323A, 323B) protrudes inwardly from the inner surface of each one of the valve blades (32A, 32B), and the scraping protrusion (323A, 323B) is a circular protrusive point.

9. The connecting base for suction catheters as claimed in claim 1, wherein
the assembling portion (21C) protrudes radially from the mounting ring edge (201C) and has
a base flange (211C) being erect and integrated with the body (20C), and having an inner side surface and an outer surrounding surface;
a surrounding ring (212C) disposed horizontally and integrated with the base flange (211C); and
four positioning ribs (213C) protruding axially from the inner side surface of the base flange (211C) and protruding toward the opening of the assembling portion (21C), the four positioning ribs (213C) respectively disposed at the 0° ,90° ,180° , and 270° positions of the base flange (211C), and the four positioning ribs (213C) formed with an inner surrounding surface of the surrounding ring (212C);
the positioning ring (31C) has
two positioning faces (311C) formed on the inner surrounding surface of the positioning ring (31 C), the position and the shape of the two positioning faces (311C) corresponding to the positions and the shapes of the two contacting faces (223C), and the two positioning faces (311C) abutting the two contacting faces (223C) of the jointing tube (22C) respectively; and
four mounting recesses (312C) radially formed in an outer surrounding surface of the positioning ring (31C), each one of the four mounting recesses (312C) axially formed through the outer surrounding surface of the positioning ring (31C), the shapes and the positions of the four mounting recesses (312C) corresponding to the shapes and positions of the four positioning ribs (213C), and the four positioning ribs (213C) engaging with the four mounting recesses (312C) respectively.

10. The connecting base for suction catheters as claimed in claim 9, wherein each one of the two valve blades (32C) has an inner surface and a scraping protrusion (323C), the scraping protrusion (323C) protrudes inwardly from the inner surface of each one of the valve blades (32C), and the scraping protrusion (323C) is a circular protrusive point.
